(19) Europäisches Patentamt
European Patent Office
Office européen des brevets

(11) Publication number: **0 066 799**
**B1**

(12) # EUROPEAN PATENT SPECIFICATION

(45) Date of publication of patent specification: **02.01.86**

(51) Int. Cl.⁴: **C 07 D 401/12,**
C 07 D 401/14, A 61 K 31/53
// C07D413/14, C07D251/18

(21) Application number: **82104660.4**

(22) Date of filing: **27.05.82**

(54) **Nicotinic acid derivatives.**

(30) Priority: **05.06.81 JP 87124/81**

(43) Date of publication of application:
**15.12.82 Bulletin 82/50**

(45) Publication of the grant of the patent:
**02.01.86 Bulletin 86/01**

(84) Designated Contracting States:
**BE CH DE FR GB IT LI**

(56) References cited:
**GB-A-1 441 904**
**GB-A-1 511 218**
**GB-A-2 078 214**

(73) Proprietor: **NIPPON SHINYAKU COMPANY, LIMITED**
**14, Kisshoin Nishinosho Monguchicho**
**Minami-ku Kyoto-shi Kyoto (JP)**

(72) Inventor: **Enomoto, Hiroshi**
**26-3-7-707 Babakemba Hashiri**
**Nagaokakyo-shi Kyoto-fu (JP)**
Inventor: **Nomura, Akira**
**2-1-1 Higashiyama Hirakata-shi**
**Osaka-fu (JP)**
Inventor: **Aoyagi, Yoshiaki**
**8-7-105 Uchidehama**
**Otsu-shi (JP)**
Inventor: **Shibata, Yoshihisa**
**1-125 Yamamoto Nakasenda**
**Kameoka-shi Kyoto-fu (JP)**

(74) Representative: **Wey, Hans-Heinrich, Dipl.-Ing. et al**
**Patentanwälte Müller-Börner & Wey**
**Widenmayerstrasse 49**
**D-8000 München 22 (DE)**

**EP 0 066 799 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel nicotinoylbenzoguanamine derivatives represented by the following general formula [I]

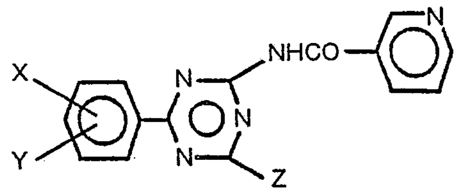

[I]

and acid addition salts thereof. In the formula, X and Y are the same or different and stand for hydrogen or halogen (excluding the case where both X and Y are hydrogens) and Z stands for mono- or di-($C_1$—$C_4$ alkyl) amino, piperidino, pyrrolidino, or morpholino.

The present inventors have extensively carried out chemical and pharmacological studies on benzoguanamine derivatives (cf. British patent 1441904, Swiss patent 596193, U.S. patent 4103009, British patent 1511218, German patent 2713933 and Japanese Kokai patent 57-35587) and, recently, have found that the compounds represented by the above general formula [I] strongly inhibit a reversed passive Arthus reaction (hereinafter abbreviated as "RPAR"). The present inventors have established technically advantageous synthetic methods of such compounds after many studies and accomplished the present invention.

Compounds according to the present invention are all basic substances and form salts with various acidic substances. Examples of salts which may be applied as pharmaceuticals are salts with hydrochloric acid, hydrobromic acid, sulfuric acid, citric acid, malonic acid, tartaric acid, malic acid, maleic acid, fumaric acid, benzoic acid, salicylic acid, cinnamic acid, methanesulfonic acid, benzene-sulfonic acid, tosylic acid, and nicotinic acid.

Preferably X and Y stand both for chlorine and Z for butylamino or diethylamino.

Other preferred groups of compounds of formula (I) are those with X is chlorine and Y is hydrogen or X is fluorine and Y is hydrogen and in the latter case Z is dimethylamino.

Substances which inhibit RPAR are useful as remedies for rheumatism and for allergic nephritis in clinical fields. Activity of RPAR inhibition of the present invention compounds were measured by the following way.

Thus, anti-BSA serum of rabbits obtained by administration of bovine serum albumin (abbreviated as BSA hereinafter) to rabbits were applied in the skin of abdomen of rats wich were previously treated with BSA and Evans Blue, then local inflammation caused by the above procedure was determined by calculating the area of the part where Evans Blue was leaked, the above data were compared with the control value, and the case where an inhibition of twenty percent or more was observed was defined as "positive". Areas of four inflammation parts per one drug to be tested were measured and the numbers of inflammation parts decided to be positive were given as X, and the inhibition activity was shown as X/4.

In the following Table 1 were shown the inhibition activities of representative compounds of the present invention by oral administration of two hundred milligrams per kilogram body weight of (1) to (10).

TABLE 1

| Compound Numbers | 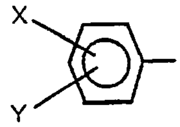 | Z | Melting Points (in centigrade) | RPAR Inhibition |
|---|---|---|---|---|
| (1) | 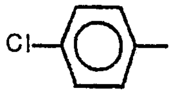 | 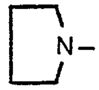 | 204~206 | 2/4 |
| (2) | 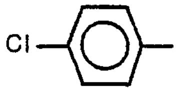 | 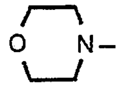 | 243~245 | 2/4 |
| (3) | 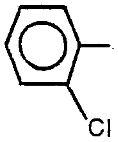 | 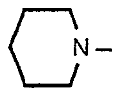 | 163~164 | 2/4 |
| (4) | 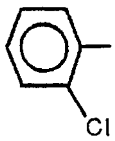 | 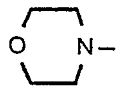 | 158~160 | 2/4 |
| (5) | 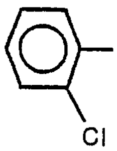 | 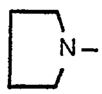 | Amorphous powder | 2/4 |
| (6) | 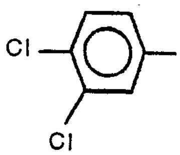 | $CH_3(CH_2)_3NH-$ | 149~153 | 2/4 |
| (7) | 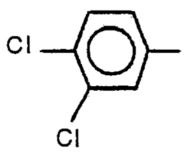 | 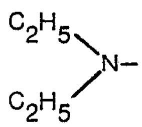 | 140~143 | 3/4 |
| (8) | 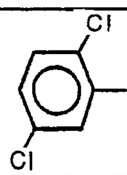 | 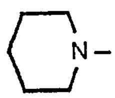 | 162~164 | 3/4 |
| (9) | 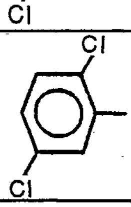 | 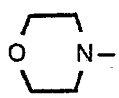 | 184~187 | 4/4 |
| (10) | 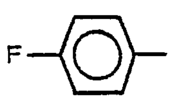 | 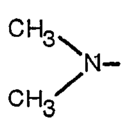 | 189~193 | 3/4 |

The RPAR inhibition activity is a characteristic pharmacological action to the present invention compounds. For example, compounds without nicotinoyl group (compounds 11 and 12) do not exhibit such an activity at all and the compounds in which both X and y are hydrogen or those in which Z is a mere amino group are with very little activity. Some examples are shown in the following Table 2.

TABLE 2

| Compound Numbers | Structure Formulas | Doses | RPAR Inhibition |
|---|---|---|---|
| (11) | 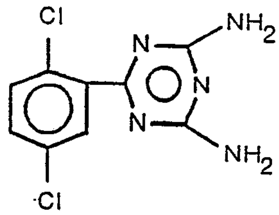 | 500 mg/kg p.o. | 0/4 |
| (12) | 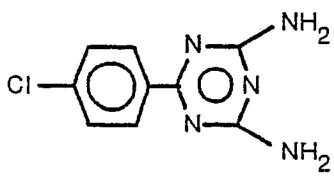 | 500 mg/kg p.o. | 0/4 |
| (13) | 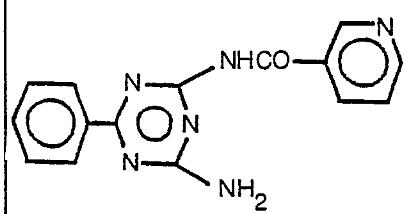 | 500 mg/kg p.o. | 2/4 |
| | | 200 mg/kg p.o. | 0/4 |
| (14) | 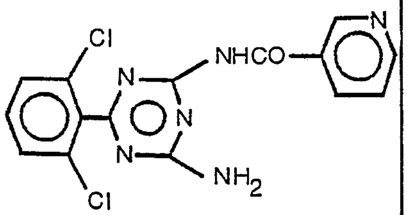 | 200 mg/kg p.o. | 0/4 |
| (15) | 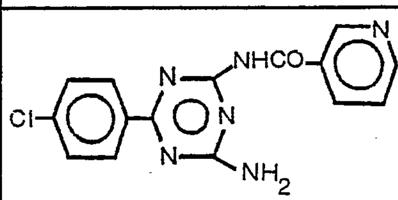 | 200 mg/kg p.o. | 0/4 |
| (16) | 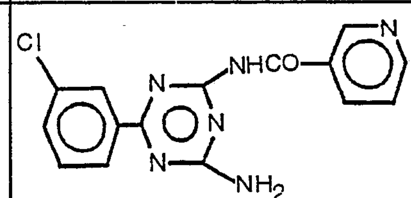 | 200 mg/kg p.o. | 0/4 |
| (17) | 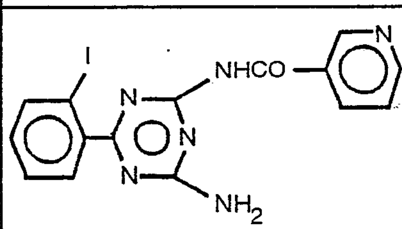 | 200 mg/kg p.o. | 1/4 |

The RPAR inhibition activity of the present invention compounds is not related with the mere antiinflammatory activity. For instance, the compound (15) exhibits 56.8 percent carrageenin edema inhibiting activity by intraperitoneal injection (50 mg/kg) but it is entirely ineffective to RPAR. On the contrary, the compound (9) strongly inhibits RPAR but its inhibition against carrageenin edema by 50 mg/kg of intraperitoneal injection is so weak as 21 percent. Further, any of phenylbutazone, indomethacin, and aspirin do not show RPAR inhibitory action by oral administration of 100, 10 and 200 mg/kg, respectively.

Acute toxicity of the compounds according to the present invention is low and, when 2000 mg/kg of any of compounds 1 to 10 is given orally to mice, no animal dies. Thus, 50 percent lethal dose is higher than 2000 mg/kg.

In case the present invention compounds are given to treat rheumatism and allergic naphritis, it is preferred to give 200 to 2000 mg to a patient daily and, more preferably, 300 to 600 mg.

Compounds of the present invention may, for example, be synthesized by the route as given the following reactions.

A) 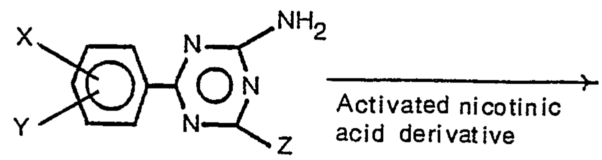

[I]

[II]

B) 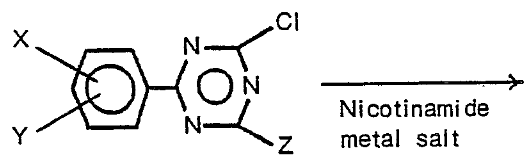

[I]

[III]

When the present invention compounds are synthesized by the route A), the starting material [II] is manufactured as follows. Thus, N-substituted biguanide derivatives (in case of the compound (1), for instance, a biguanide derivative obtained by heating pyrrolidine hydrochloride with dicyandiamide at 100 to 200°C) are heated with *p*-chlorobenzonitrile in the presence of potassium hydroxide catalyst.

The compound [II] may also be prepared by as follows. Thus, 2,4-dichloro-6-substituted phenyl-s-triazine is treated with ammonia and substituted amine successively.

Examples of activated nicotinic acid derivatives used in the method A) will be as follows. Thus, nicotinic acid anhydride, nicotinic acid chloride, dichlorophosphoric acid anhydride of nicotinic acid, sulfuric acid anhydride of nicotinic acid, sulfonic acid anhydride of nicotinic acid, and the like and such commonly-used various kinds of activated derivatives can be advantageously used.

When the synthesis is conducted in accordance with the route B), 2,4-dichloro-6-substituted phenyl-s-triazines are as first made to react with equimolar substituted amines (for example, n-butylamine in case the compound (6) is aimed) in the presence of acid removing agent and the resulting substance (III) is made to react with nicotinamide sodium salt (prepared from nicotinamide and sodium hydroxide) to give the product.

In addition to the above-mentioned compounds the invention includes the following substances:

2-(3,4-dichlorophenyl)-4-nicotinoyl-6-pyrrolidino-*s*-triazine
2-(3,4-dichlorophenyl)-4-morpholino-6-nicotinoyl-*s*-triazine
2-(2-chlorophenyl)-4-morpholino-6-nicotinoyl-*s*-triazine
2-(2-chlorophenyl)-4-nicotinoyl-6-pyrrolidino-*s*-triazine
2-butylamino-4-(3,4-dichlorophenyl)-6-nicotinoyl-*s*-triazine
2-(3,4-dichlorophenyl)-4-diethylamino-6-nicotinoyl-*s*-triazine
2-(2,5-dichlorophenyl)-4-morpholino-6-nicotinoyl-*s*-triazine
2-(4-fluorophenyl)-4-dimethylamino-6-nicotinoyl-*s*-triazine

Examples illustrating manufacture method of the present invention compounds follow:

Example 1

Synthesis of the compounds (1) and (2).

2-Amino-4-chloro-6-(4-chlorophenyl)-s-triazine (melting point 273 to 275°C) (2.12 grams) and 2.5 grams of pyrrolidine were dissolved in 20 milliliters of dioxane, the mixture was heated to reflux for two hours with stirring, the reaction solution was evaporated to dryness in vacuo, diluted aqueous solution of sodium hydroxide was added to the residue, insoluble matters were collected by filtration, and washed with isopropyl alcohol to give 2.21 grams of 2-amino-4-pyrrolidino-6-(4-chlorophenyl)-s-triazine, melting point 217 to 219°C.

The product obtained here (2.0 grams) was heated to reflux for three hours in 20 milliliters of dioxane together with 1.8 grams of nicotinic acid anhydride. The reaction solution was evaporated to dryness in vacuo and the residue was washed with water and recrystallized from isopropyl alcohol and dioxane mixture to give 1.84 grams of the substance (1). Melting point 204 to 206°C.

When the same reaction was conducted using morpholine instead of pyrrolidine, the compound (2) was obtained.

Example 2

Synthesis of the compounds (3), (4), (5) and (10)

Dicyandiamide (8.4 grams) and piperidine hydrochloride (12.1 grams) were heated at 145 to 150°C for four hours in 20 milliliters of methylcellosolve. After cooled, the reaction solution was mixed with 6.8 grams of sodium ethoxide and 30 milliliters of methylcellosolve, then heated to reflux for four hours with 15 grams of methyl *o*-chlorobenzoate, cooled, diluted with 200 milliliters of water, the crystals separated out therefrom were collected by filtration, and washed with methanol to give 6.3 grams of 2-amino-4-piperidino-6-(2-chlorophenyl)-s-triazine, melting point 184 to 186°C.

The resulting product (3.6 grams) and 4.6 grams of nicotinic acid anhidride were heated to reflux with stirring for four hours. The reaction solution was evaporated to dryness in vacuo, the residue was dissolved in chloroform, washed with 1 percent aqueous solution of potassium carbonate, the solvent was evaporated therefrom, the residue was dissolved in benzene with warming, and cooled to give crystals which were recrystallized from benzene to give 4.35 grams of the substance (3), melting point 163 to 164°C.

When the same reaction was carried out using morpholine hydrochloride or pyrrolidine hydrochloride instead of piperidine hydrochloride, compounds (4) and (5), respectively, were obtained. When the reaction with dicyandiamide was conducted using dimethylamine hydrochloride and the succeeding reaction was done using *p*-fluorobenzonitrile, then the compound (10) was obtained.

Example 3

Synthesis of the compounds (6) and (7)

2-Amino-4-chloro-6-(3,4-dichlorophenyl)-s-triazine (melting point 249 to 250°C) (2.75 grams) and 2.0 grams of n-butylamine were heated to reflux with stirring for four hours in 25 milliliters of dioxane. The reaction solution was evaporated to dryness in vacuo, the residue was washed with water and then with methanol, and 2.51 grams of 2-amino-4-butylamino-6-(3,4-dichlorophenyl)-s-triazine was obtained. Melting point 176 to 179°C.

Nicotinic acid (2.5 grams) was added to 10 milliliters of pyridine, 2.7 grams of isobutyl chlorocarbonate was dropped thereinto with ice-cooling and stirring within five minutes, the mixture was stirred for another thirty minutes, then 2.0 grams of the compound as obtained hereinabove was added thereto, and the mixture was stirred under refluxing for three hours. Pyridine was evaporated therefrom under reduced pressure, the residue was dissolved in methanol with warming, and allowed to stand whereupon crystals came out. They were removed by filtration, the mother liquor was concentrated, the resulting sirup was allowed to stand to give crystals. Recrystallization from methanol gave 0.91 gram of the compound (6). Melting point 149 to 153°C.

The same reaction as above was conducted using diethylamine instead of n-butylamine to give 2-amino-4-diethylamino-6-(3,4-dichlorophenyl)-s-triazine, melting point 201 to 204°C. The resulting product (2.0 grams) and 2.5 grams of nicotinic acid were added to 50 milliliters of pyridine, 2.4 grams of methanesulfonyl chloride was dropped thereinto during ten minutes at room temperature with stirring, and the mixture was heated to reflux for three hours. The reaction solution was evaporated to dryness in vacuo, to the residue was added water, insoluble matters were collected by filtration, and they were washed with methanol and recrystallized form isopropyl alcohol to give 1.25 grams of the substance (7), melting point 140 to 143°C.

Example 4

Synthesis of the compounds (8) and (9)

2,4-Dichloro-6-(2,5-dichlorophenyl)-s-triazine (3.0 grams) was dissolved in 50 milliliters of ether and, with stirring and ice cooling, 1.70 grams of piperidine was dropped thereinto during five minutes. After the mixture was stirred for one hour the reaction solution was washed with diluted hydrochloric acid and then with water and evaporated. The residual crystalline substance was used in the succeeding steps without further purification.

Nicotinamide (2.5 grams) was added to 30 milliliters of tetrahydrofuran (anhydrous), 2.0 grams of sodium hydride (50 percent) was added thereto, the mixture was stirred with refluxing for thirty minutes, cooled, the reaction product as obtained hereinabove was added to this tetrahydrofuran solution, and the mixture was further heated to reflux for three hours. The reaction solution was evaporated to dryness in vacuo, the residue was treated with chloroform to remove insoluble matters, the chloroform-soluble matters were treated with ether, and the crystalline substance obtained therefrom was recrystallized from isopropyl alcohol to give 0.87 grams of the substance (8). Melting point 162 to 164°C.

When the same reaction was conducted by the use of morpholine instead of piperidine, the compound (9) melting at 184 to 187°C was obtained.

Example 5

Synthesis of the compounds (8) and (9)

2-Amino-4-chloro-6-(2,5-dichlorophenyl)-s-triazine (1.3 grams) and 2.0 grams of piperazine were stirred with refluxing for two hours in 20 milliliters of dioxane. The reaction solution was evaporated to dryness in vacuo, water was added to the residue, crystals thereby separated out were collected by filtration, and washed with methanol to give 1.47 grams of 2-amino-4-piperazino-6-(2,5-dichlorophenyl)-s-triazine, melting at 194 to 195°C.

The product obtained hereinabove (1.70 grams) and 3.00 grams of nicotinic acid anhydride were heated to reflux for 3.5 hours in 20 milliliters of dioxane. The reaction solution was cooled, crystals separated out therefrom were discarded by filtration, the mother liquor was evaporated to dryness in vacuo, 50 percent aqueous methanol was added to the residue, crystals separated out therefrom were collected by filtration, and recrystallized from isopropyl alcohol to give 1.04 grams of the substance (8), melting point 162 to 164°C.

When the same reaction was carried out by the use of morpholine instead of piperidine, the substance (9) was obtained. Melting point 184 to 187°C.

**Claims**

1. Nicotinoylbenzoguanamine derivatives, and acid addition salts thereof, represented by the following general formula [I]

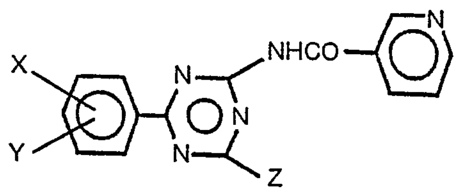

[I]

in which X and Y may be the same or different and they stand for hydrogen or halogen (excluding the case where both X and Y are hydrogens) and Z stands for mono- or di($C_1$—$C_4$ alkyl) amino, piperidino, pyrrolidino or morpholino.

2. Compounds according to Claim 1 in which both X and Y are chlorines.
3. Compounds according to Claim 2 in which Z is butylamino.
4. Compounds according to Claim 2 in which Z is diethylamino.
5. Compounds according to Claim 1 in which X is chlorine and Y is hydrogen.
6. Compounds according to Claim 1 in which X is fluorine and Y is hydrogen.
7. Compounds according to Claim 6 in which Z is dimethylamino.
8. Antiallergic agents containing nicotinoylbenzoguanamine derivatives having the general formula (I) and/or acid addition salts thereof according to claims 1 to 7.

**Patentansprüche**

1. Nicotinoylbenzoguanaminderivate und ihre Säureadditionssalze der folgenden allgemeinen Formel (I)

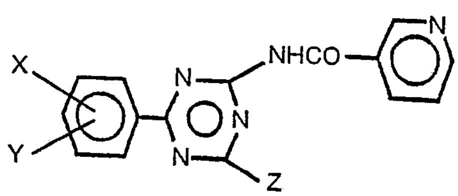

[I]

in der X und Y gleich oder unterschiedlich und ein Wasserstoff- oder Halogenatom sind (mit der Ausnahme des Falls, wo sowohl X als auch Y Wasserstoffatome sind) und Z eine Mono- oder Di-($C_1$—$C_4$-alkyl)amino-, Piperidino-, Pyrrolidino- oder Morpholinogruppe ist.

2. Verbindungen nach Anspruch 1, wobei sowohl X als auch Y Chloratome sind.
3. Verbindungen nach Anspruch 2, wobei Z die Butylaminogruppe ist.
4. Verbindungen nach Anspruch 2, wobei Z die Diethylaminogruppe ist.
5. Verbindungen nach Anspruch 1, wobei X ein Chloratom und Y ein Wasserstoffatom ist.
6. Verbindungen nach Anspruch 1, wobei X ein Fluoratom und Y ein Wasserstoffatom ist.
7. Verbindungen nach Anspruch 6, wobei Z die Dimethylaminogruppe ist.
8. Antiallergische Mittel, enthaltend Nicotinoylbenzoguanaminderivate der allgemeinen Formel (I) und/oder ihre Säureadditionssalze nach einem der Ansprüche 1 bis 7.

**Revendications**

1. Dérivés de nicotinoylbenzoquanamine, et leurs sels d'addition d'acides, représentés par la formule générale [I] suivante

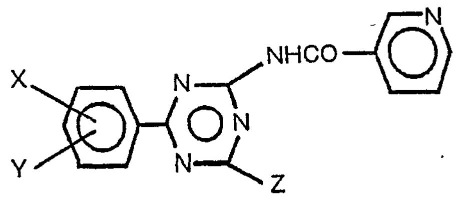

[I]

dans laquelle X et Y peuvent être identiques ou différents et représentent l'hydrogène ou un halogène (le cas où X et Y sont tous deux H étant exclu) et Z représente un groupe mono- ou di-(alkyl en $C_1$—$C_4$) amino, pipéridino, pyrrolidino ou morpholino.

2. Composés selon la revendication 1 dans lesquels X et Y sont tous deux Cl.

3. Composés selon la revendication 2 dans lesquels X est butylamino.

4. Composés selon la revendication 2 dans lesquels Z est diéthylamino.

5. Composés selon la revendication 1 dans lesquels X est Cl et Y est H.

6. Composés selon la revendication 1 dans lesquels X est F et Y est H.

7. Composés selon la revendication 6 dans lesquels Z est diméthylamino.

8. Agents antiallergiques contenant des dérivés de nicotinoylbenzoguanamine répondant à la formule générale [I] et/ou leurs sels d'addition d'acide selon les revendications 1 à 7.